# EUROPEAN PATENT SPECIFICATION

(11) **EP 2 709 706 B1**
(45) Date of publication and mention of the grant of the patent: **24.06.2015**
(21) Application number: 12723357.5
(22) Date of filing: 18.05.2012
(51) Int. Cl.: A61M 25/00, A61M 25/10, A61M 25/01

(54) **BALLOON CATHETER WITH IMPROVED PUSHABILITY**
BALLONKATHETER MIT VERBESSERTER VORSCHIEBBARKEIT
CATHÉTER À BALLONNET AVEC POUSSABILITÉ AMÉLIORÉE

(30) Priority: 20.05.2011 US 201161488579 P
(43) Date of publication of application: 26.03.2014
(73) Proprietor: Boston Scientific Scimed, Inc., Maple Grove, MN 55311 (US)
(72) Inventor: GROVENDER, Adam D., Brooklyn Park, Minnesota 55444 (US); JOHNSON, Jeffry, Plymouth, Minnesota 55446 (US); SPITAEL, Pieter, New Brighton, Minnesota 55112 (US)
(74) Representative: Vossius & Partner Patentanwälte Rechtsanwälte mbB
(86) International application number: PCT/US2012/038701
(87) International publication number: WO 2012/162186

(56) References cited:
- EP-A1- 1 787 673
- WO-A1-92/07610
- WO-A1-93/18816
- WO-A2-2011/011765

## Description

### Cross-Reference to Related Application

This application claims priority under 35 U.S.C. §119 to U.S. Provisional Application Serial No. 61/488,579, filed May 20, 2011.

### Technical Field

The present invention relates generally to catheters for performing medical procedures. More particularly, the present invention relates to balloon catheters.

### Background

A wide variety of intracorporeal medical devices have been developed for medical use, for example, intravascular use. Some of these devices include guidewires, catheters, and the like. These devices are manufactured by any one of a variety of different manufacturing methods and may be used according to any one of a variety of methods. Of the known medical devices and methods, each has certain advantages and disadvantages. Examples of medical devices are found in WO 92/07610, EP 1787673 or WO 2011/011765. There is an ongoing need to provide alternative medical devices as well as alternative methods for manufacturing and using medical devices.

### Brief Summary

The invention provides a balloon catheter as disclosed in claim 1. An example medical device may include a balloon catheter. An example balloon catheter may include a proximal shaft. A midshaft may be attached to the proximal shaft. A distal shaft may be attached to the midshaft. A balloon may be coupled to the distal shaft. An inflation lumen may be defined that extends from the proximal shaft, through the midshaft, and into the distal shaft. The inflation lumen may be in fluid communication with the balloon. A core wire may extend through a portion of the inflation lumen. The midshaft may define an interior ridge along a portion of the inflation lumen. The core wire may have a shoulder that abuts the interior ridge of the midshaft.

Another example balloon catheter may include a catheter shaft having a proximal shaft portion, a midshaft portion attached to the proximal shaft portion, and a distal shaft portion attached to the midshaft portion. A balloon may be coupled to the catheter shaft. A guidewire port may be formed in the midshaft portion. The guidewire port may be in fluid communication with a guidewire lumen extending along a portion of the catheter shaft. An inflation lumen may be defined in the catheter shaft. The inflation lumen may be in fluid communication with the balloon. A core wire may extend through the inflation lumen. The midshaft portion may have an interior ridge formed therein and positioned adjacent to the inflation lumen. The core wire may have a shoulder that contacts the interior ridge of the midshaft.

An example method for manufacturing a balloon catheter may include providing a catheter shaft having an inflation lumen extending therethrough and disposing a mandrel in the inflation lumen. The mandrel may have a first stepped shoulder formed therein. The method may also include heating the catheter shaft so that a portion of the catheter shaft changes in shape so as to have an interior ridge that is complimentary in shape to the first stepped shoulder and providing a core wire. The core wire may have a second stepped shoulder. The method may also include placing the core wire within the inflation lumen so that the second stepped shoulder abuts the interior ridge.

The above summary of some embodiments is not intended to describe each disclosed embodiment or every implementation of the present invention. The Figures, and Detailed Description, which follow, more particularly exemplify these embodiments.

### Brief Description of the Drawings

The invention may be more completely understood in consideration of the following detailed description of various embodiments of the invention in connection with the accompanying drawings, in which:
Figure 1 is a plan view of an example balloon catheter;
Figure 2 is a cross-sectional view of a portion of the example balloon catheter shown in Figure 1;
Figure 3 is a cross-sectional view taken through line 3-3 in Figure 2;
Figures 4-9 illustrate some of the example method steps for manufacturing the balloon catheter shown in Figure 1-3.
Figure 10 is a cross-sectional side view of an example core wire;
Figure 11 illustrates a portion of an example catheter shaft having the core wire shown in Figure 10 disposed therein;
Figure 12 is a cross-sectional view taken through line 12-12 in Figure 11; and
Figure 13 is a cross-sectional side view of another example core wire.

### Detailed Description

For the following defined terms, these definitions shall be applied, unless a different definition is given in the claims or elsewhere in this specification.

All numeric values are herein assumed to be modified by the term "about," whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

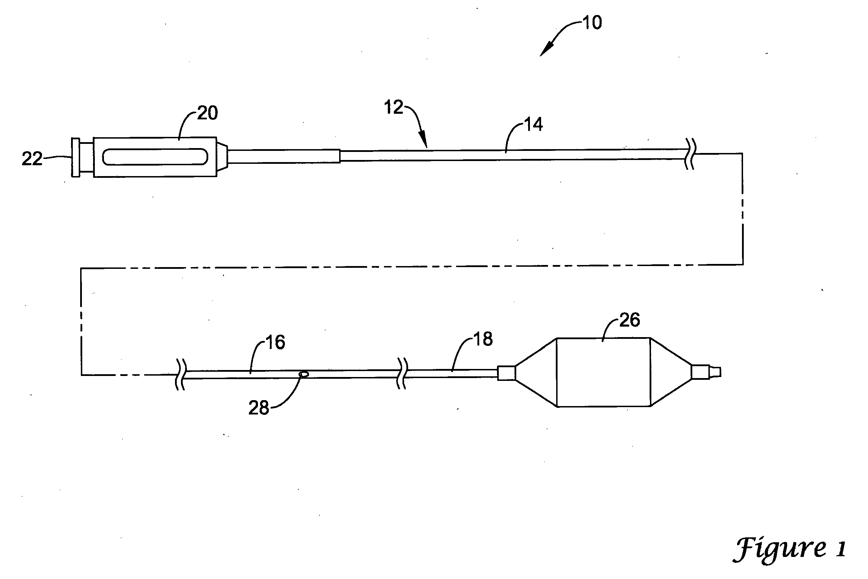

Figure 1 is a plan view of an example catheter 10, for example a balloon catheter. Catheter 10 may include a catheter shaft 12 having a proximal shaft portion, 14, a midshaft portion 16 and a distal shaft portion 18. In some examples proximal shaft portion 14 may be a metallic hypotube. Midshaft portion 16 may be fitted over, fitted within, or abut proximal shaft portion 14, as appropriate. Likewise, distal shaft portion 18 may be fitted over, fitted within, or abut midshaft portion 16. These are just examples as any suitable arrangement may be utilized. A hub 20 may be attached to proximal shaft portion 14. Hub 20 may include one or more ports such as, for example, a port 22.

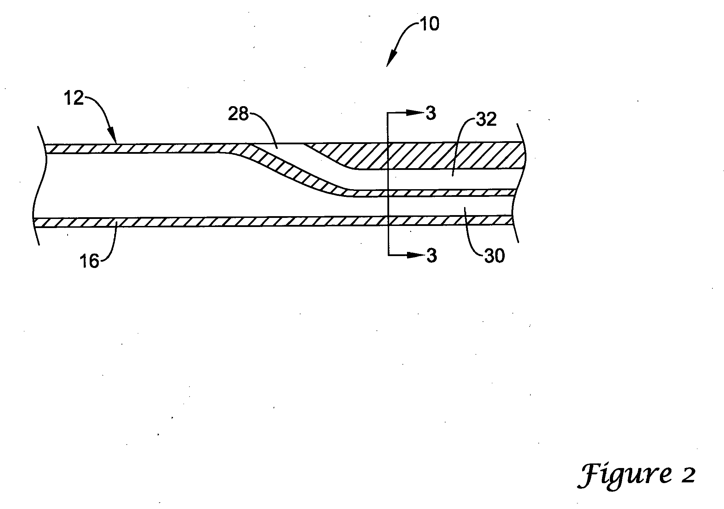

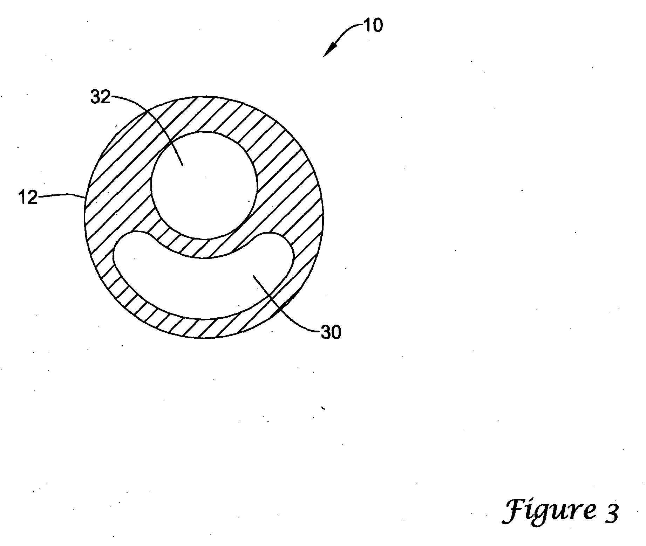

An expandable balloon 26 may be attached to distal shaft portion 18. Balloon 26 may be expanded by infusing inflation media through an inflation lumen 30, which is shown in Figure 2. In at least some examples, port 22 may provide access to inflation lumen 30. Accordingly, a suitable inflation device may be attached to port 22 and inflation media may be passed through inflation lumen 30 to inflate balloon 26. Along a region of midshaft portion 16, inflation lumen 30 may have an annular shape as seen in Figure 3. This may be due to the formation of a guidewire port 28 in midshaft portion 16. Some additional details regarding the formation of guidewire port 28 and/or inflation lumen 30 are provided herein.

As indicated above, guidewire port 28 may be formed in midshaft portion 16. For example, guidewire port 28 may be an opening extending through the wall of midshaft portion 16 that provides access to a guidewire lumen 32. In the example depicted in Figure 2, guidewire port 28 is positioned at a location that is distal to the proximal end of catheter shaft 12. When so arranged, catheter 10 may be a single-operator-exchange or rapid-exchange catheter, which allows catheter 10 to be used with a shorter guidewire. As such, guidewire lumen 32 may extend over only a portion of the length of catheter shaft 12. For example, guidewire lumen 32 may extend along distal shaft portion 18 and part of midshaft portion 16. Other examples, however, are contemplated where catheter 10 is an over-the-wire catheter or fixed wire catheter. In these examples, guidewire lumen 32 may extend along essentially the entire length of catheter shaft 12.

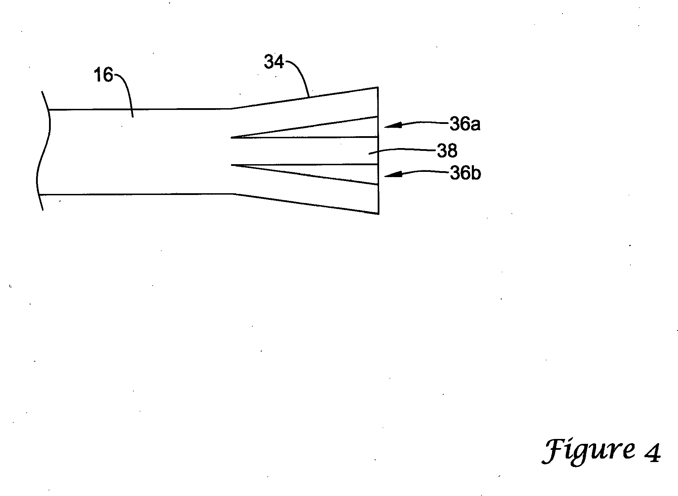

Figures 4-9 illustrate some of the processing steps that may be utilized to form catheter 10 and/or catheter shaft 12. For example, Figure 4 shows part of midshaft portion 16. Here it can be seen that a distal end 34 of midshaft portion 16 may be flared or otherwise enlarged. In addition, one or more cuts or slots, for example cuts 36a/36b, may be formed in distal end 34 of midshaft portion 16. A tongue 38 may be defined between cuts 36a/36b.

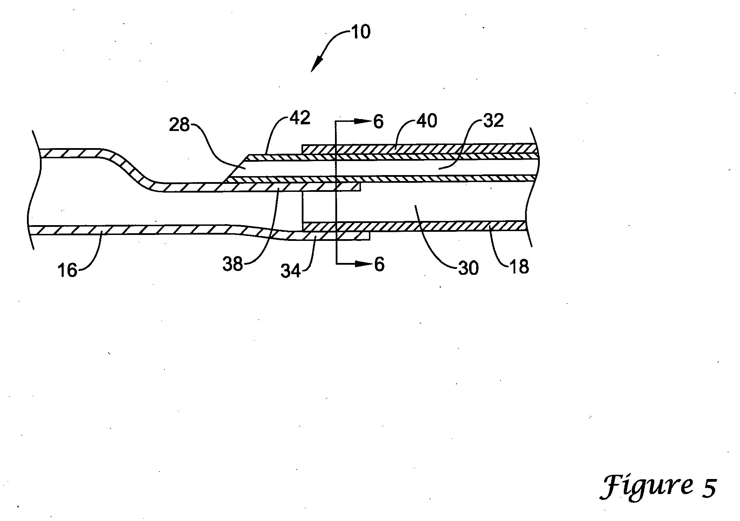

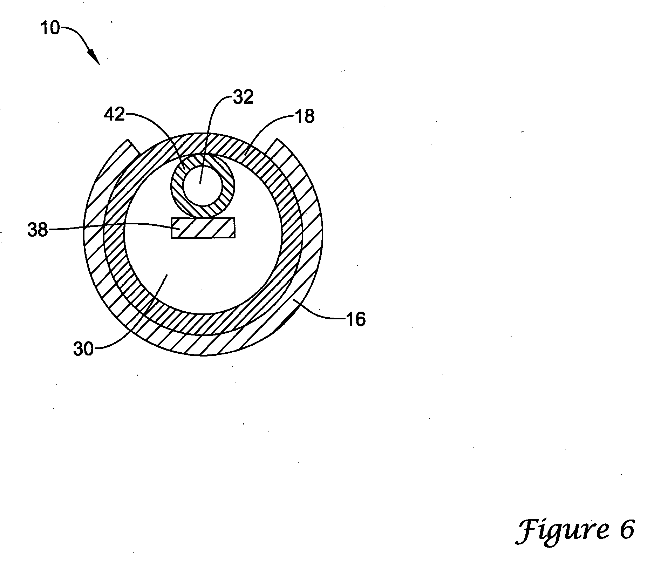

A proximal end 40 of distal shaft portion 18 may be disposed within the enlarged distal end 34 of midshaft portion 16 as shown in Figure 5. In doing so, tongue 38 may be pressed inward and form a shelf or ledge. This may created or define a guidewire ramp in catheter shaft 12 adjacent to guidewire port 28. A distal inner tube 42 may be disposed within distal shaft portion 18 and may rest upon the ledge formed by tongue 38. Distal inner tube 42 may ultimately form guidewire lumen 32 as described in more detail below. The arrangement of distal inner tube 42 relative to tongue 38, midshaft portion 16, and distal shaft portion 18 can also be seen in Figure 6.

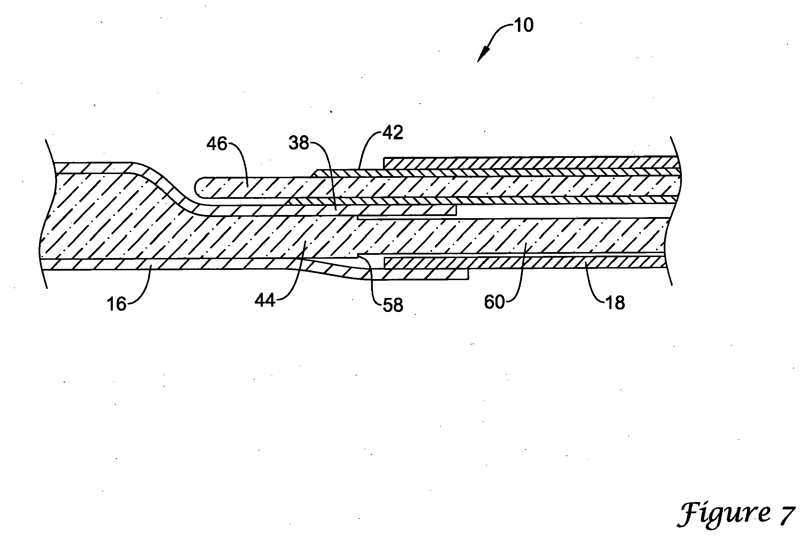

When suitably arranged, a first mandrel 44 may be inserted within a portion of distal shaft portion 18 and midshaft portion 16 as shown in Figure 7. Likewise, a second mandrel 46 may be inserted within distal inner tube 42. Mandrels 44/46 are generally configured to maintain lumens 30/32 when catheter shaft 12 is subjected to heat and/or further processing as described in more detail below.

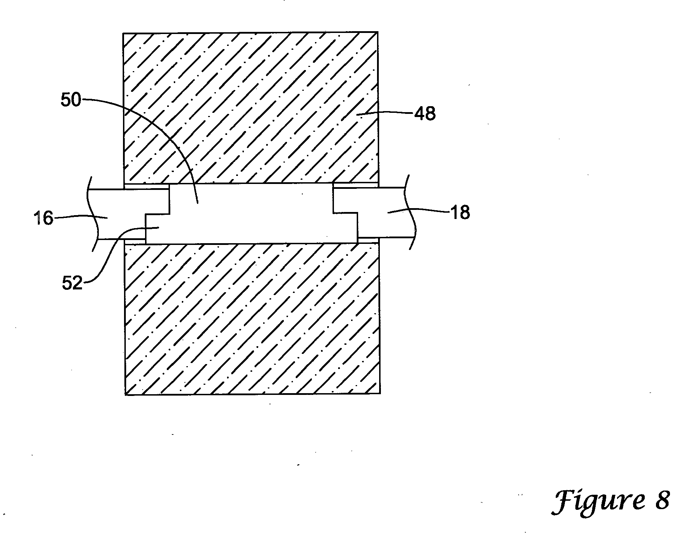

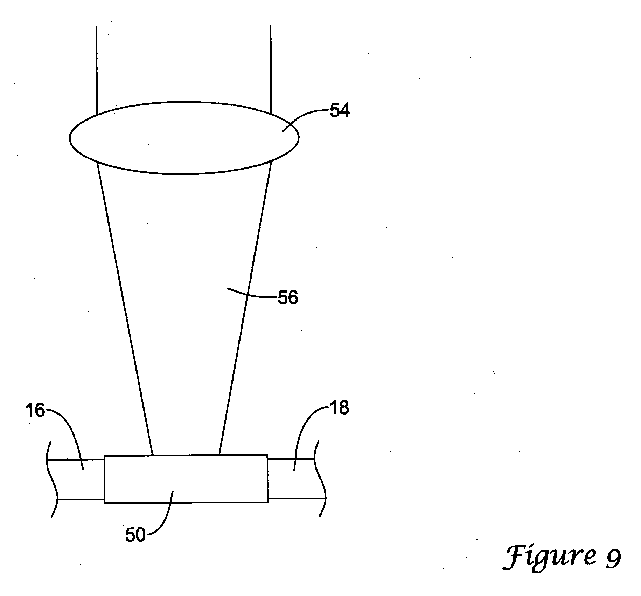

With mandrels 44/46 in place, midshaft portion 16 and distal shaft portion 18 may be disposed within a compression fixture 48 as shown in Figure 8. A sleeve 50 may be disposed over a region of midshaft portion 16 and distal shaft portion 18. Sleeve 50 may include one or more flanking ears 52, which may aid in removal of sleeve 50 upon completion of the manufacturing process. Finally, heat may be applied to sleeve 50. This may include the use of a lens 54 to focus heat (e.g., laser energy 56) onto sleeve 50 as depicted in Figure 9. When heated, midshaft portion 16, distal shaft portion 18, and distal inner tube 42 may melt together. Mandrels 44/46 can be removed, thereby defining inflation lumen 30 and guidewire lumen 32, respectively, and the result may be the formation of catheter shaft 12 as shown herein.

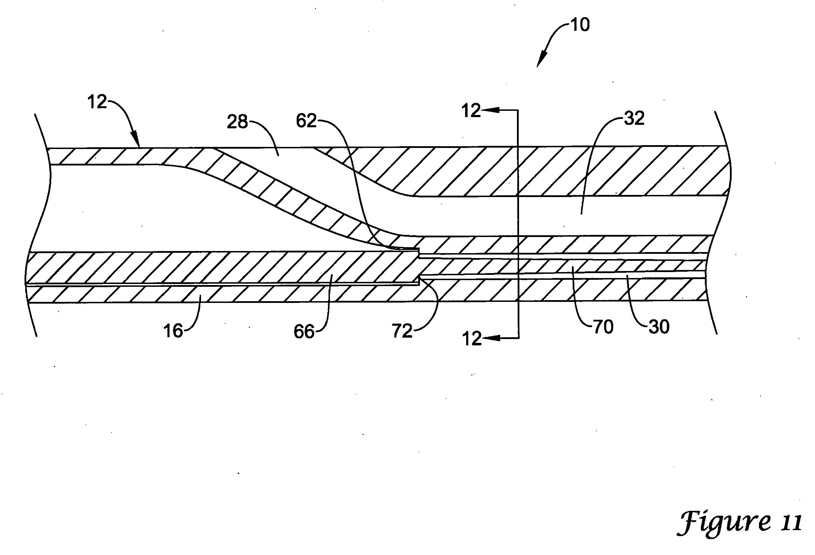

Referring back to Figure 7, mandrel 44 may include a stepped shoulder 58 and a distal section 60. Stepped shoulder 58 may be configured to create a ridge or shelf 62 (not shown in Figures 2 or 7, but can be seen in Figure 11) along the interior of catheter shaft 12 when catheter shaft 12 is subjected to the heating and/or compressing steps disclosed herein. In at least some examples, this interior ridge 62 is disposed along midshaft portion 16. However, the interior ridge 62 can be disposed at other locations along the length of catheter shaft 12.

The shape or configuration of ridge 62 may be similar to or complimentary to the stepped shoulder 58 of mandrel 44. For example, stepped shoulder 58 may take the form of a substantially stepwise change in the outer diameter of mandrel 44 such that ridge 62 has a corresponding stepped shape. Other examples are contemplated, however, where stepped shoulder 58 has a different shape so that the shape of ridge 62 is also different. For example, shoulder 58 may have be tapered and/or sloped, include more than one step or changes in outer diameter, may include projections or ridge, or have any other suitable configuration. It can be appreciated that regardless of the shape of shoulder 58, ridge 62 is configured to have a corresponding or complimentary shape.

Catheters like catheter 10 may be designed to have increased or increasing distal flexibility. This may be desirable because portions of the catheter 10, particularly distal portions, may need to navigate sharp bends or turns within the vasculature. Because of this, however, it may be challenging to push the catheter through the vasculature in a reliable manner. In other words, increased distal flexibility, while being desirable for allowing the catheter to navigate the tortuous anatomy, may make it more difficult to "push" the catheter through the anatomy.

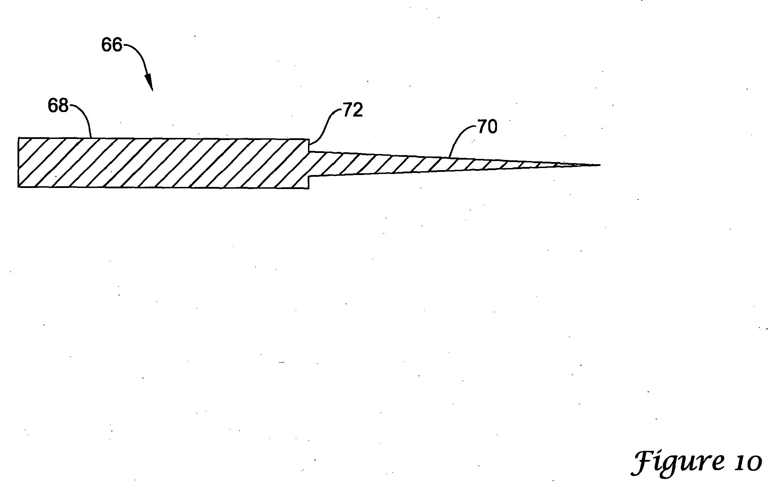

In order to improve the pushability of catheter 10, a core wire 66, which can be seen in Figure 10, may be disposed within catheter shaft 12. Core wire 66 may generally take the form of a wire or rod. In some examples, core wire 66 may have a substantially uniform outer diameter or dimension. In other examples, core wire 66 may include one or more tapers or other changes in outer diameter. For example, core wire 66 may include a proximal section 68 having a substantially uniform outer diameter and a distal section 70 that is tapered.. This may be desirable for a number of reasons. For example, tapering distal section 70 may allow for a gradual transition in flexibility along portions of the length of catheter shaft 12 (e.g., at or near transitions between portions 14/16/18). In addition, core wire 66 may be a singular wire having a solid cross-section. In other examples, core wire 66 may be tubular or include portions that are tubular. In still other examples, core wire 66 may include a plurality of wire filaments that may be longitudinally aligned, twisted, braided, or the like.

Core wire 66 may extend from proximal shaft portion 14, across midshaft portion 16, and into distal shaft portion 18. For example, proximal section 68 may extend along proximal shaft portion 14 (and, in some examples, along part of midshaft portion 16), shoulder 70 may be disposed at midshaft portion 16, and distal section 70 may extend into distal shaft portion 18. These are just examples as other configurations are contemplated.

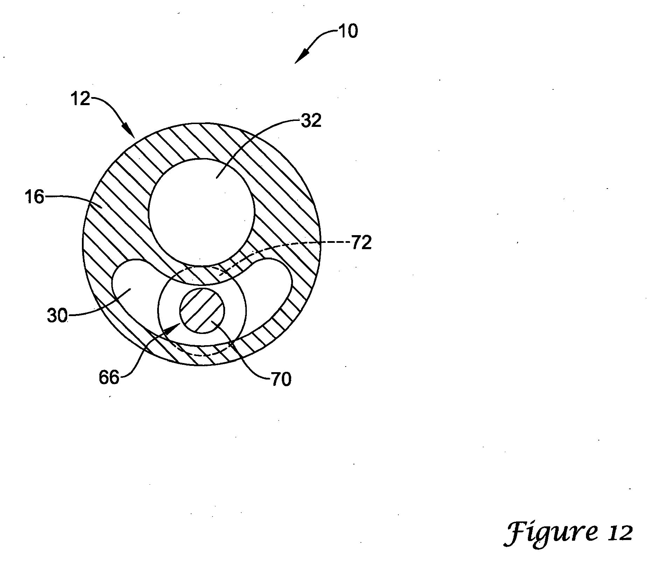

Core wire 66 may include a shoulder 72. In general, shoulder 72 is configured to abut ridge 62 formed in catheter shaft 12 (e.g., along the interior of midshaft portion 16) as seen in Figures 11-12. This arrangement may be desirable for a number of reasons. For example, the abutting arrangement may allow forces applied at the proximal end of catheter shaft 12 to be transferred more efficiently to more distal portions of catheter shaft 12 such as along midshaft portion 16, adjacent to guidewire port 28, along distal shaft portion 18, or other suitable locations. Because, for example, push forces can be efficiently transferred, the abutting arrangement may desirably impact the pushability of catheter 10. In general, it may be desirable for ridge 62 to be disposed along midshaft portion 16, for example adjacent to guidewire port 28 so that core wire 66 can abut ridge 62 at this location. This may allow for push forces to be desirably transferred along catheter shaft 12 at positions adjacent to guidewire port 28. Other positions for ridge 62 are contemplated including other portions of midshaft portion 16, along proximal shaft portion 14, and along distal shaft portion 18.

For the purposes of this disclosure, abutting may be understood to mean that direct physical contact at the ends of two or more structures. Accordingly, the abutting relationship of shoulder 72 and ridge 62 may be understood to mean that an end of shoulder 72 comes into direct physical contact with ridge 62. In other words, the ends of shoulder 72 and ridge 62 contact one another in an abutting manner. This may aid in the transfer of forces, for example push forces, from core wire 66 to midshaft portion 16 and/or other portions of catheter shaft 12. In some examples, shoulder 72 may also be attached to ridge 62. This may include an adhesive bond, a thermal bond, a laser bond, or other suitable bonds.

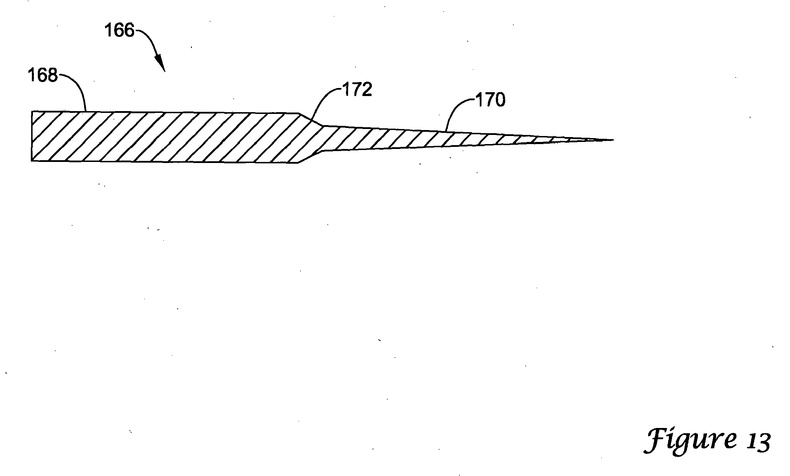

In some examples, shoulder 72 is a stepped shoulder or change in outer diameter as depicted in Figures 11-12. This, however, is not intended to be limiting as other examples are contemplated where transitions other than a stepped change may be utilized. For example, Figure 13 illustrates another example core wire 166, which may be similar in form and function to core wire 166. Core wire 166 includes proximal section 168 and distal section 170. Between sections 168/170, is a tapered or sloped shoulder region 172. Other configurations and shapes are also contemplated for shoulders formed on core wires.

The materials that can be used for the various components of catheter 10 may include those commonly associated with medical devices. For simplicity purposes, the following discussion makes reference to catheter shaft 12 and other components of catheter 10. However, this is not intended to limit the devices and methods described herein, as the discussion may be applied to other similar tubular members and/or components of tubular members or devices disclosed herein.

Catheter shaft 12 and/or other components of catheter 10 may be made from a metal, metal alloy, polymer (some examples of which are disclosed below), a metal-polymer composite, ceramics, combinations thereof, and the like, or other suitable material. Some examples of suitable metals and metal alloys include stainless steel, such as 304V, 304L, and 316LV stainless steel; mild steel; nickel-titanium alloy such as linear-elastic and/or super-elastic nitinol; other nickel alloys such as nickel-chromium-molybdenum alloys (e.g., UNS: N06625 such as INCONEL® 625, UNS: N06022 such as HASTELLOY® C-22®, UNS: N10276 such as HASTELLOY® C276®, other HASTELLOY® alloys, and the like), nickel-copper alloys (e.g., UNS: N04400 such as MONEL® 400, NICKELVAC® 400, NICORROS® 400, and the like), nickel-cobalt-chromium-molybdenum alloys (e.g., UNS: R30035 such as MP35-N® and the like), nickel-molybdenum alloys (e.g., UNS: N10665 such as HASTELLOY® ALLOY B2®), other nickel-chromium alloys, other nickel-molybdenum alloys, other nickel-cobalt alloys, other nickel-iron alloys, other nickel-copper alloys, other nickel-tungsten or tungsten alloys, and the like; cobalt-chromium alloys; cobalt-chromium-molybdenum alloys (e.g., UNS: R30003 such as ELGILOY®, PHYNOX®, and the like); platinum enriched stainless steel; titanium; combinations thereof; and the like; or any other suitable material.

As alluded to herein, within the family of commercially available nickel-titanium or nitinol alloys, is a category designated "linear elastic" or "non-super-elastic" which, although may be similar in chemistry to conventional shape memory and super elastic varieties, may exhibit distinct and useful mechanical properties. Linear elastic and/or non-super-elastic nitinol may be distinguished from super elastic nitinol in that the linear elastic and/or non-super-elastic nitinol does not display a substantial "superelastic plateau" or "flag region" in its stress/strain curve like super elastic nitinol does. Instead, in the linear elastic and/or non-super-elastic nitinol, as recoverable strain increases, the stress continues to increase in a substantially linear, or a somewhat, but not necessarily entirely linear relationship until plastic deformation begins or at least in a relationship that is more linear that the super elastic plateau and/or flag region that may be seen with super elastic nitinol. Thus, for the purposes of this disclosure linear elastic and/or non-super-elastic nitinol may also be termed "substantially" linear elastic and/or non-super-elastic nitinol.

In some cases, linear elastic and/or non-super-elastic nitinol may also be distinguishable from super elastic nitinol in that linear elastic and/or non-super-elastic nitinol may accept up to about 2-5% strain while remaining substantially elastic (e.g., before plastically deforming) whereas super elastic nitinol may accept up to about 8% strain before plastically deforming. Both of these materials can be distinguished from other linear elastic materials such as stainless steel (that can also can be distinguished based on its composition), which may accept only about 0.2 to 0.44 percent strain before plastically deforming.

In some examples, the linear elastic and/or non-super-elastic nickel-titanium alloy is an alloy that does not show any martensite/austenite phase changes that are detectable by differential scanning calorimetry (DSC) and dynamic metal thermal analysis (DMTA) analysis over a large temperature range. For example, in some examples, there may be no martensite/austenite phase changes detectable by DSC and DMTA analysis in the range of about -60 degrees Celsius (°C) to about 120 °C in the linear elastic and/or non-super-elastic nickel-titanium alloy. The mechanical bending properties of such material may therefore be generally inert to the effect of temperature over this very broad range of temperature. In some examples, the mechanical bending properties of the linear elastic and/or non-super-elastic nickel-titanium alloy at ambient or room temperature are substantially the same as the mechanical properties at body temperature, for example, in that they do not display a super-elastic plateau and/or flag region. In other words, across a broad temperature range, the linear elastic and/or non-super-elastic nickel-titanium alloy maintains its linear elastic and/or non-super-elastic characteristics and/or properties.

In some examples, the linear elastic and/or non-super-elastic nickel-titanium alloy may be in the range of about 50 to about 60 weight percent nickel, with the remainder being essentially titanium. In some examples, the composition is in the range of about 54 to about 57 weight percent nickel. One example of a suitable nickel-titanium alloy is FHP-NT alloy commercially available from Furukawa Techno Material Co. of Kanagawa, Japan. Some examples of nickel titanium alloys are disclosed in U.S. Patent Nos. 5,238,004 and 6,508,803, which are incorporated herein by reference. Other suitable materials may include ULTANIUM™ (available from Neo-Metrics) and GUM METAL™ (available from Toyota). In some other examples, a superelastic alloy, for example a superelastic nitinol can be used to achieve desired properties.

In at least some examples, portions or all of catheter shaft 12 may also be doped with, made of, or otherwise include a radiopaque material. Radiopaque materials are understood to be materials capable of producing a relatively bright image on a fluoroscopy screen or another imaging technique during a medical procedure. This relatively bright image aids the user of catheter 10 in determining its location. Some examples of radiopaque materials can include, but are not limited to, gold, platinum, palladium, tantalum, tungsten alloy, polymer material loaded with a radiopaque filler, and the like. Additionally, other radiopaque marker bands and/or coils may also be incorporated into the design of catheter 10 to achieve the same result.

In some examples a degree of Magnetic Resonance Imaging (MRI) compatibility is imparted into catheter 10. For example, catheter shaft 12, or portions thereof, may be made of a material that does not substantially distort the image and create substantial artifacts (i.e., gaps in the image). Certain ferromagnetic materials, for example, may not be suitable because they may create artifacts in an MRI image. Catheter shaft 12, or portions thereof, may also be made from a material that the MRI machine can image. Some materials that exhibit these characteristics include, for example, tungsten, cobalt-chromium-molybdenum alloys (e.g., UNS: R30003 such as ELGILOY®, PHYNOX®, and the like), nickel-cobalt-chromium-molybdenum alloys (e.g., UNS: R30035 such as MP35-N® and the like), nitinol, and the like, and others.

A sheath or covering (not shown) may be disposed over portions or all of catheter shaft 12 that may define a generally smooth outer surface for catheter 10. In other examples, however, such a sheath or covering may be absent from a portion of all of catheter 10, such that catheter shaft 12 may form the outer surface. The sheath may be made from a polymer or other suitable material. Some examples of suitable polymers may include polytetrafluoroethylene (PTFE), ethylene tetrafluoroethylene (ETFE), fluorinated ethylene propylene (FEP), polyoxymethylene (POM, for example, DELRIN® available from DuPont), polyether block ester, polyurethane (for example, Polyurethane 85A), polypropylene (PP), polyvinylchloride (PVC), polyether-ester (for example, ARNITEL® available from DSM Engineering Plastics), ether or ester based copolymers (for example, butylene/poly(alkylene ether) phthalate and/or other polyester elastomers such as HYTREL® available from DuPont), polyamide (for example, DURETHAN® available from Bayer or CRISTAMID® available from Elf Atochem), elastomeric polyamides, block polyamide/ethers, polyether block amide (PEBA, for example available under the trade name PEBAX®), ethylene vinyl acetate copolymers (EVA), silicones, polyethylene (PE), Marlex high-density polyethylene, Marlex low-density polyethylene, linear low density polyethylene (for example REXELL®), polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide (for example, KEVLAR®), polysulfone, nylon, nylon-12 (such as GRILAMID® available from EMS American Grilon), perfluoro(propyl vinyl ether) (PFA), ethylene vinyl alcohol, polyolefin, polystyrene, epoxy, polyvinylidene chloride (PVdC), poly(styrene-*b*-isobutylene-*b-*styrene) (for example, SIBS and/or SIBS 50A), polycarbonates, ionomers, biocompatible polymers, other suitable materials, or mixtures, combinations, copolymers thereof, polymer/metal composites, and the like. In some examples the sheath can be blended with a liquid crystal polymer (LCP). For example, the mixture can contain up to about 6 percent LCP.

In some examples, the exterior surface of the catheter 10 (including, for example, the exterior surface of catheter shaft 12) may be sandblasted, beadblasted, sodium bicarbonate-blasted, electropolished, etc. In these as well as in some other examples, a coating, for example a lubricious, a hydrophilic, a protective, or other type of coating may be applied over portions or all of the sheath, or in examples without a sheath over portion of catheter shaft 12, or other portions of catheter 10. Alternatively, the sheath may comprise a lubricious, hydrophilic, protective, or other type of coating. Hydrophobic coatings such as fluoropolymers provide a dry lubricity which improves guidewire handling and device exchanges. Lubricious coatings improve steerability and improve lesion crossing capability. Suitable lubricious polymers are well known in the art and may include silicone and the like, hydrophilic polymers such as high-density polyethylene (HDPE), polytetrafluoroethylene (PTFE), polyarylene oxides, polyvinylpyrolidones, polyvinylalcohols, hydroxy alkyl cellulosics, algins, saccharides, caprolactones, and the like, and mixtures and combinations thereof. Hydrophilic polymers may be blended among themselves or with formulated amounts of water insoluble compounds (including some polymers) to yield coatings with suitable lubricity, bonding, and solubility. Some other examples of such coatings and materials and methods used to create such coatings can be found in U.S. Patent Nos. 6,139,510 and 5,772,609, which are incorporated herein by reference.

The coating and/or sheath may be formed, for example, by coating, extrusion, co-extrusion, interrupted layer co-extrusion (ILC), or fusing several segments end-to-end. The layer may have a uniform stiffness or a gradual reduction in stiffness from the proximal end to the distal end thereof. The gradual reduction in stiffness may be continuous as by ILC or may be stepped as by fusing together separate extruded tubular segments. The outer layer may be impregnated with a radiopaque filler material to facilitate radiographic visualization.

## Claims

1. A balloon catheter (10), comprising:
a catheter shaft (12) having a proximal shaft portion (14), a midshaft portion (16) attached to the proximal shaft portion, and a distal shaft portion (18) attached to the midshaft portion;
a balloon (26) coupled to the catheter shaft;
wherein a guidewire port (28) is formed in the midshaft portion, the guidewire port being in fluid communication with a guidewire lumen (32) extending along a portion of the catheter shaft;
wherein an inflation lumen (30) is defined in the catheter shaft, the inflation lumen being in fluid communication with the balloon;
a core wire (66) extending through the inflation lumen;
**characterised in that**:
the midshaft portion has an interior stepped ridge (62) formed therein and positioned adjacent to the inflation lumen; and
the core wire has a stepped shoulder (72) that contacts the interior stepped ridge of the midshaft.

2. The balloon catheter of claim 1, wherein the core wire has a distal region (70) extending distally from the shoulder.

3. The balloon catheter of claim 2, wherein the distal region is tapered and extends into the distal shaft portion.

4. The balloon catheter of claim 1, wherein a guidewire ramp is formed in the midshaft adjacent to the guidewire port.

## Patentansprüche

1. Ballonkatheter (10), der aufweist:
einen Katheterschaft (12) mit einem proximalen Schaftabschnitt (14), einem Mittelschaftabschnitt (16), der am proximalen Schaftabschnitt befestigt ist, und einem distalen Schaftabschnitt (18), der am Mittelschaftabschnitt befestigt ist;
einen Ballon (26), der mit dem Katheterschaft gekoppelt ist;
wobei ein Führungsdrahtport (28) im Mittelschaftabschnitt gebildet ist, wobei der Führungsdrahtport in Fluidverbindung mit einem Führungsdrahtlumen (32) steht, das sich entlang eines Abschnitts des Katheterschafts erstreckt;
wobei ein Inflationslumen (30) im Katheterschaft gebildet ist, wobei das Inflationslumen in Fluidverbindung mit dem Ballon steht;
einen Kerndraht (66), der sich durch das Inflationslumen erstreckt;
**dadurch gekennzeichnet, dass**:
der Mittelschaftabschnitt eine innere abgestufte Leiste (62) hat, die darin gebildet und benachbart zum Inflationslumen positioniert ist; und
der Kerndraht eine abgestufte Schulter (72) hat, die die innere abgestufte Leiste des Mittelschafts kontaktiert.

2. Ballonkatheter nach Anspruch 1, wobei der Kerndraht einen distalen Bereich (70) hat, der sich distal von der Schulter erstreckt.

3. Ballonkatheter nach Anspruch 2, wobei der distale Bereich zulaufend ist und sich in den distalen Schaftabschnitt erstreckt.

4. Ballonkatheter nach Anspruch 1, wobei eine Führungsdrahtrampe im Mittelschaft benachbart zum Führungsdrahtport gebildet ist.

## Revendications

1. Cathéter à ballonnet (10) comprenant :
une tige de cathéter (12) ayant une partie de tige proximale (14) et une partie de tige centrale (16) fixée à la partie de tige proximale, et une partie de tige distale (18) fixée à la partie de tige centrale ;
un ballonnet (26) couplé à la tige de cathéter ;
dans lequel un orifice de fil-guide (28) est formé dans la partie de tige centrale, l'orifice de fil-guide étant en communication de fluide avec une lumière de fil-guide (32) s'étendant le long d'une partie de la tige de cathéter ;
dans lequel une lumière de gonflage (30) est définie dans la tige de cathéter, la lumière de gonflage étant en communication de fluide avec le ballonnet ;
une tige d'armature (66) s'étendant à travers la lumière de gonflage ;
**caractérisé en ce que** :
la partie de tige centrale a une crête étagée intérieure (62) formée à l'intérieur de cette dernière et positionnée de manière adjacente à la lumière de gonflage ; et
la tige d'armature a un épaulement étagé (72) qui est en contact avec la crête étagée intérieure de la tige centrale.

2. Cathéter à ballonnet selon la revendication 1, dans lequel la tige d'armature a une région distale (70) s'étendant de manière distale à partir de l'épaulement.

3. Cathéter à ballonnet selon la revendication 2, dans lequel la région distale est progressivement rétrécie et s'étend dans la partie de tige distale.

4. Cathéter à ballonnet selon la revendication 1, dans lequel une rampe de fil-guide est formée dans la tige centrale adjacente à l'orifice de fil-guide.
